# EUROPEAN PATENT SPECIFICATION

(11) **EP 0 599 525 B1**
(45) Date of publication and mention of the grant of the patent: **21.07.1999**
(21) Application number: 93309096.1
(22) Date of filing: 15.11.1993
(51) Int. Cl.: G01N 1/22, G01N 33/00

(54) **Gas sensor**
Gassensor
Capteur de gaz

(30) Priority: 25.11.1992 GB 9224677
(43) Date of publication of application: 01.06.1994
(73) Proprietor: EDINBURGH SENSORS LIMITED, Edinburgh EH14 4AP (GB)
(72) Inventor: Bramley, Paul, Kirknewton, Midlothian, EH27 8EE (GB); Shaw, Brian, Corstorphine, Edinburgh, EH12 8PZ (GB)
(74) Representative: Rackham, Stephen Neil

(56) References cited:
- EP-A- 0 503 327
- FR-A- 2 564 624
- GB-A- 2 197 077
- GB-A- 2 237 518
- US-A- 4 115 229
- US-A- 4 605 855
- PATENT ABSTRACTS OF JAPAN vol. 13, no. 587 (P-983) 25 December 1989 & JP-A-01 250 753 (TOKYO ELECTRIC POWER CO INC) 05 October 1989
- PATENT ABSTRACTS OF JAPAN vol. 10, no. 290 (P-503)2 October 1986 & JP-A-61 108 947 (FUJI ELECTRIC CORP RES AND DEV LTD) 27 May 1986
- PATENT ABSTRACTS OF JAPAN vol. 16, no. 159 (P-1339) 17 April 1992 & JP-A-04 009 735 (HITACHI CABLE LTD) 14 January 1992
- PATENT ABSTRACTS OF JAPAN vol. 9, no. 274 (E-354)31 October 1985 & JP-A-60 117 702 (FUJI DENKI SOUGOU KENKYUSHO KK) 25 June 1985

## Description

The present invention relates to a sensor for detecting the presence of gas in an environment. The system may be used, for example, with an infra-red gas sensor, but also is applicable to other types of sensor.

An infra-red absorption gas sensor works by passing a beam of infra-red radiation through a sample of the gas. The power received on the other side of the sample from the infra-red source is measured at a wavelength which is characteristically absorbed by a particular component gas. If the component in question is not present, then the signal received is a maximum. As the concentration of the gas component increases, the signal received decreases and the extent of the decrease gives a measure of the amount of the component present in the sample.

In such a system, the gas sensor normally includes a sample cell and it is necessary to aspirate the sensor, that is to take a sample of the gas from the environment and to transfer it into the sample volume contained within the cell. Hitherto this has been done either by using a pump to draw a sample from the environment into the cell, or alternatively by allowing diffusion to transfer the sample from the environment to the sampling volume. A diffusion gas cell may have holes in the enclosure defining the sampling volume in order to ease diffusion and minimise the response time. Even so, the response time of diffusion gas cells tends to be very slow compared with pumped units. Pump systems however have disadvantages of their own. In particular, the bulk transfer of gas from the environment into the cell makes the sensor vulnerable to damage resulting from the ingress of dust or water. An example of a pump system is disclosed in GB-A-2,237,518.

It has previously been proposed to provide a gas sensor in which a diffusion surface is mounted directly above the sensing element in a common housing. Gas is heated in the limited space outside the diffusion surface to promote the flow of gas through the surface. One example of such a device is disclosed in FR-A-2564624. However, the diffusion speeds attainable with such devices have been found to be undesirably low. The diffusion surface is necessarily small in area and it is difficult to establish a convection flow with any efficiency simply by heating the very limited volume of gas on the outer side of the diffusion surface.

JP-A-1250753 discloses a sensor system which uses a cylindrical metallic filter to introduce gas into a sensor cell. Again, the size of the filter is limited because of the need to match it to the size of the sensor, and the functioning of this system depends upon gas being positively forced into the sensor under pressure.

An alternative apparatus is disclosed in US-A-4,115,229. In this system the sample gas is itself heated causing the gas to flow directly past a sensor element.

According to the present invention there is provided a gas sensor comprising a diffusion surface and a sensing element, the sensing element being housed separately from the diffusion surface and linked to the diffusion surface by a duct, characterised in that the duct provides a convection path for the aspiration of the sensing element by a convection current carrying gas from the diffusion surface.

The present invention uses a combination of convection and diffusion to aspirate the sensor in a gas cell. This is done using a structure in which the sensor and diffuser are separated and linked by a convection duct. Unlike prior art systems, convection is not used to drive gas across a diffusion surface, but rather to carry the gas from the diffusion surface to the sensor. It is found that by doing this response times are obtained which are comparable with pump systems, while avoiding all the disadvantages associated with the bulk transfer of gas into a sample volume. In addition a sensor formed in this manner has a lower power consumption than a pumped unit, is considerably more reliable in operation, and is quieter.

Preferably the cell comprises means for heating gas in a region of the duct, so as to set up the convection current.

Preferably the duct is formed as a closed loop.

In the preferred embodiment of the invention, gas in the duct is warmed to set up convection currents. These currents carry gas from the diffusion surface in contact with the environment to the sensor. The diffusion surface as well as allowing gas exchange to take place with the environment, also operates as the outflow heat exchanger for the convection current.

Preferably the diffusion surface is formed as a tube making up one arm of the duct.

The use of a diffusion tube provides a large surface area and makes it possible for the process of heat-exchange to be completed by the time the convection current has carried the gas along the length of the tube.

Preferably the diffusion surface is formed from a micro-porous plastics material such as polyethylene or PTFE.

Preferably the sensor is an infra-red absorption sensor, and the infra-red source is arranged also to provide the said means for heating the gas.

Alternatively, or in addition, a separate heater may be formed around a portion of the duct.

A fan may be provided in the duct to provide a forced convection flow.

The use of a fan speeds up the response time of the cell after power up and ensures that the cell can be used in any desired orientation.

Although described in relation to an infra-red sensor, the system may also be used with other sensors such as pellistor devices or semiconductor sensors. The invention may also be used in liquid sensors where the fluid diffused from the environment is a liquid rather than a gas.

Examples of gas sensors in accordance with the present invention will now be described in detail and contrasted with the prior art with reference to the accompanying drawings, in which:
Figure 1 is a diagram showing a conventional infra-red sensor;
Figure 2 is a side elevation of a first example of a gas sensor in accordance with the present invention;
Figure 3 is a side elevation of a second example;
Figure 4 is a side elevation of a third example incorporating a fan;
Figure 5 is a side elevation of a fourth example using a pellistor sensor; and
Figure 6 is an exploded perspective view showing in detail an embodiment of the gas sensor.

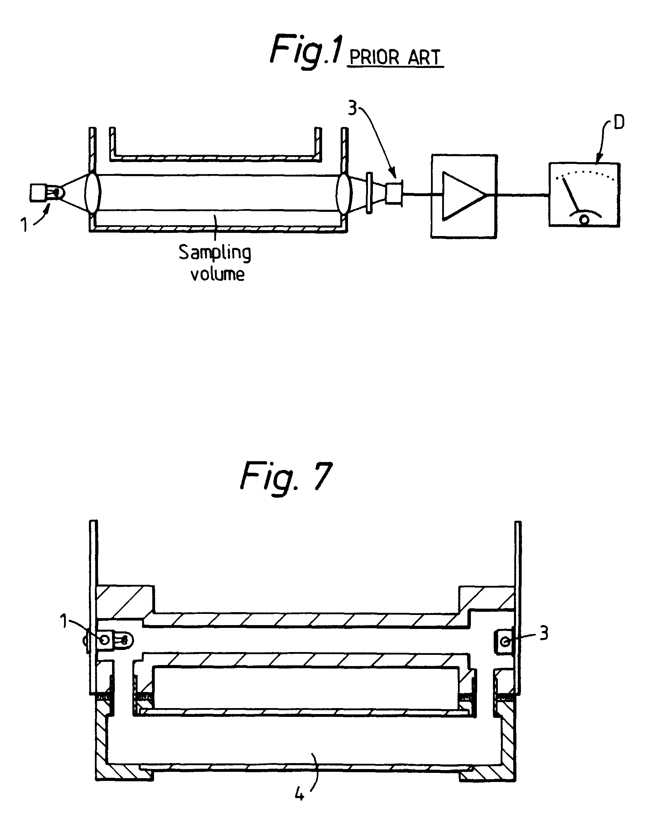

Figure 1 shows a conventional infra-red gas sensor. As described above, either a sampling pump or a diffusion surface is used to introduce gas into a sampling volume. An infra-red source is positioned at one end of the sampling volume and a detector at the other. A wavelength selection filter tuned to the characteristic wavelength of the selected gas component is placed before the detector. The output of the detector is processed to provide a display D indicating the concentration of the selected gas component.

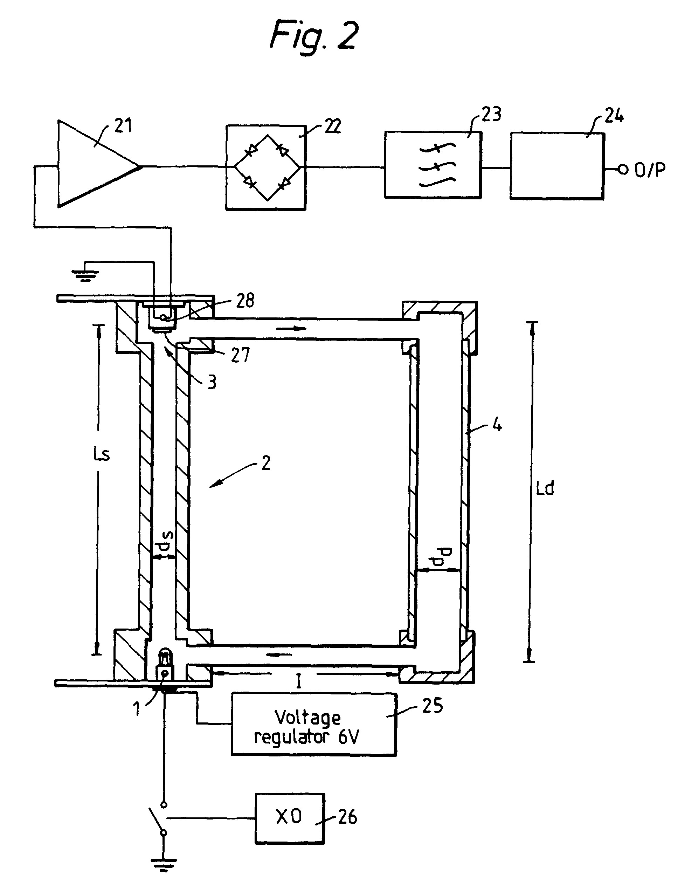

Figure 2 shows a first example of an infra-red gas sensor in accordance with the present invention. An infra-red source 1 is positioned at the bottom of one arm of a closed-loop duct 2. A infra-red detector 3 is mounted opposite the source and operates in the manner described above in relation to Figure 1. A tube 4 of micro-porous polyethylene with a pore size of approximately 70 microns is formed in the other arm of the duct. Gas from the environment in which the cell is placed flows into the tube 4 and heat is output into the environment also across the tube 4.

Figure 2 also shows in block diagram form the control and power circuits for the infra-red gas sensor. The sensor and the associated circuits may be generally conventional in nature. The infra-red source 1 is powered via a 6 volt voltage regulator 25 and a crystal oscillator 26 operating at 8Hz. The sensing element 3 comprises an infra-red filter 27 and a pyroelectric detector 28. The output from the detector 28 is taken to a preamplifier 21. The amplified signal is then rectified in a precision rectifier 22 and passed through a low pass filter 23 before being processed in a signal processor 24 to produce an output in the form suitable for display or recordal.

The excess power from the IR source 1 heats the gas in the duct causing a local decrease in density. This warmer gas is then displaced by the cooler and more dense gas flowing in from the diffusion tube 4 under gravity. The warmer gas from the sampling volume is pushed into the top of the diffusion cell where it cools and falls. In this way, a closed loop convection system is set up in which the gas flows relatively quickly around the loop formed by the sampling volume, the diffusion cell and the interconnecting ducts.

The material of the diffusion tube 4 is hydrophobic and has a sufficiently small pore size to prevent water or dust reaching the sensor.

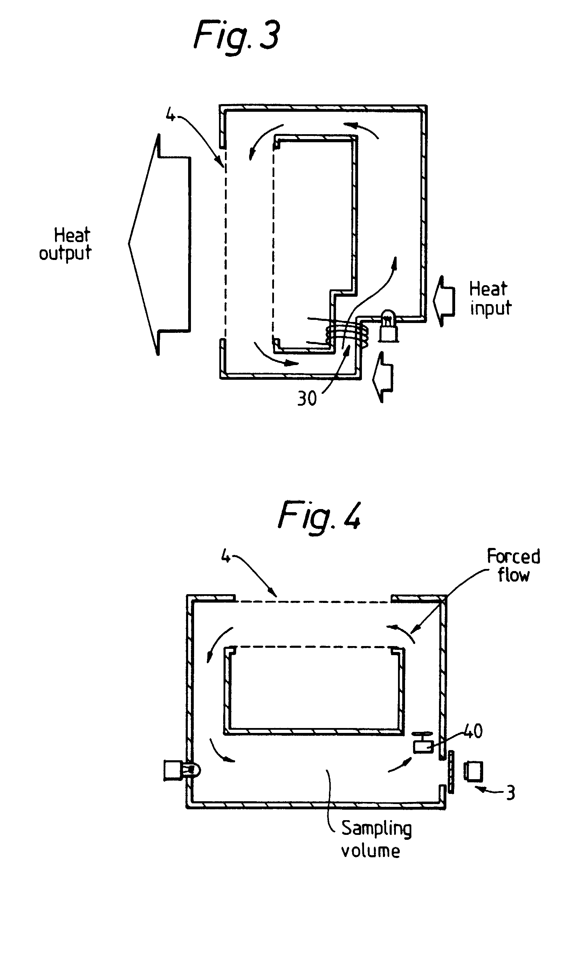

With such a system it is found that the response time is proportional to the power input. The response time can be further enhanced by adding a heater 50 in series with the cell as shown in Figure 3.

Normally the convection current takes several minutes to become established and relies upon the cell being oriented with the diffusion tube and the sampling volume in the vertical plane with the infra-red source at the bottom. For systems which must operate in any orientation, or systems which must operate rapidly after power up, a fan 40 may be added inside the duct to force the flow of gas, as shown in Figure 4.

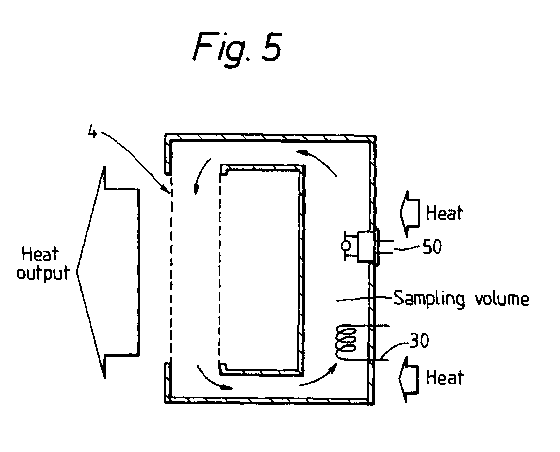

Figure 5 shows an alternative embodiment using a pellistor gas sensor 50 in place of an infra-red sensor. A heating element 30 is again included to enhance the convection flow.

It is found that the sensor shown in Figure 2 achieves a response time (to 90% of change) of 50s which is comparable with the response time of 30s of a conventional pumped system using a one litre per minute pump. By adding a 1W heater, as shown in Figure 3, the response time can be reduced to less than 30s.

Although it is much preferred to use a closed loop, other arrangements for a convection-aspirated sensor are possible. For example, appropriately located baffles might be positioned within an open cell to encourage the desired convection currents.

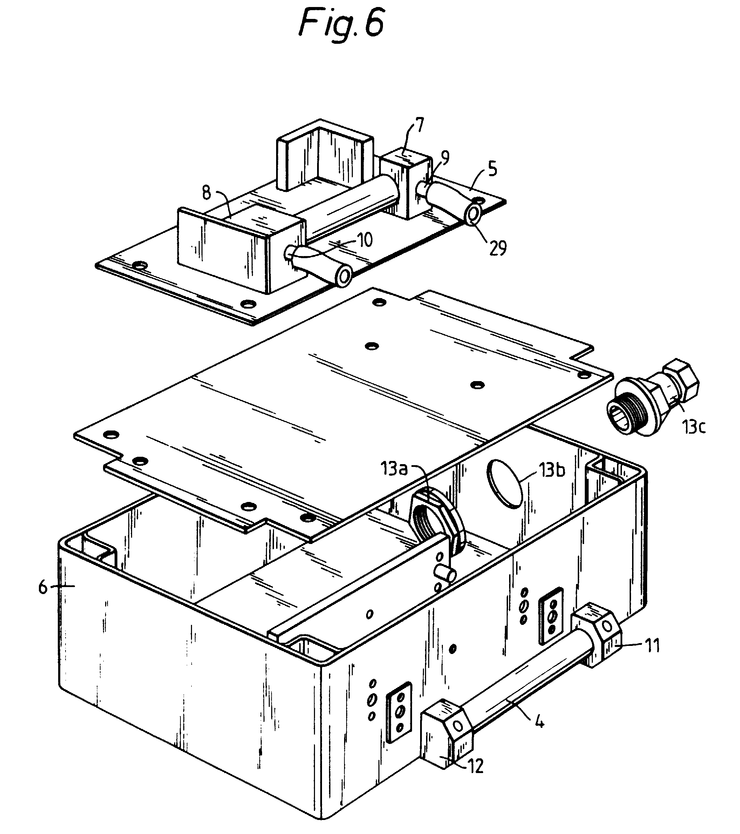

Figure 6a shows in detail an embodiment generally corresponding to the type of layout shown in Figure 2. In this embodiment, the arm of the system incorporating the infra-red source 1 is mounted on a circuit board 5 within a casing 6. This arm of the duct is machined from an aluminium alloy as a hollow cylinder and includes a cuboid steel housing 7, 8 at each end. One of these cuboid housings contains the infra-red source, and the other the infra-red detector. Rigid cylindrical ports 9, 10 extending from the respective housings 7, 8 link the housings to neoprene rubber tubes 29. These extend through the casing to make a gas connection with the diffusion tube 4. The diffusion tube 4 is fixed to the exterior of the casing by mountings 11, 12. These support the diffusion tube 4 away from the side of the casing so that gas in the environment of the sensor system can flow freely around the diffusion tube 4. A port 13a, 13b, 13c provides for the entry into the casing of cables carrying a power supply to the gas sensor and also carrying output sensor data.

Figure 7 shows another embodiment, in which the diffusion tube 4 and sample cell are rigidly mechanically coupled together, and the side-arms of the duct are much shorter by comparison with the embodiment of Figure 6.

In the embodiment of Figure 2, the dimensions of the major components are as set out in table 2. Most of the dimensions illustrated can be varied whilst still providing an operative system. It is found however that if the internal diameters of components of the ducts such as the tubes connecting the diffusion cell to the sample cell are less than 3mm then the performance of the system is poor unless additional heating is used to enhance the convection flow. It is much preferred that the internal diameter for all the components making up the duct, that is the sample cell, diffusion cell and the tubes linking the two, should be greater than or equal to 5mm. It is also preferred that the diffusion and sample cells should be significantly longer than they are wide. One of these two cells may be shortened provided that the other remains long. In this case the pipe connecting the two parts takes over as one of the "chimneys". Generally all the dimensions of the components may be increased so long as one arm of the system retains the chimney-like dimensions (i.e. is much longer than it is wide). It will be seen that for the embodiment of Figure 2 the ratio of diffusion tube length to internal diameter is 7:1. In alternative embodiments this ratio may vary but preferably is greater than substantially 3:1, and more preferably greater than substantially 5:1. Similar constraints apply to the dimensions of the sample cell.

Table 1 below shows below shows experimental results obtained with gas sensors in accordance with the present invention. In example b, which falls outside the scope of the present invention but is included for comparison, a zero convection condition is induced by having the sensor tube and diffusion tube horizontal and at the same height so that any temperature gradient is developed in the horizontal plane and being perpendicular to the gravitational force produces no convection flow around the loop. For the examples shown in Table 1, the diffusion cell and sample cell were linked by tubes of approximately 6 mm internal diameter.

**TABLE 1**

| | | | | |
|---|---|---|---|---|
| | Aspiration Method | Flow Generation | Optical HeadLength | Response Time (T90) |
| a | Diffusion | Convection Vertically oriented cells No extra heating | 100 mm | 48 seconds |
| b | Diffusion | None | 100 mm | 8-10 minutes |
| c | Diffusion | Convection Horizontal cells diffusion uppermost | | 1 minute - 50 seconds |
| d | Diffusion | Convection & boost heater, 1W | 100 mm | 24 seconds |

**TABLE 2**

| | mm |
|---|---|
| Sample Tube Length | |
| Lₛ | 105 |
| Diffusion Tube Length | 105 |
| L_{d} | |
| Sample Tube I.D. | 8 |
| dₛ | |
| Diffusion Tube I.D. | 15 |
| d_{D} | |
| Interconnect Length | 63 |
| I | |
| Interconnect I.D. | 6 |

## Claims

1. A gas sensor comprising a diffusion surface (4) and a sensing element (1,3), the sensing element (1,3) being housed separately from the diffusion surface (4) and linked to the diffusion surface (4) by a duct (2), characterised in that the duct provides a convection path for the aspiration of the sensing element (1,3) by a convection current carrying gas from the diffusion surface (4).

2. A gas sensor according to claim 1, in which the duct is formed as a closed loop.

3. A sensor according to claim 2, in which the diffusion surface extends along one arm of the duct, and the sensing element is located in another arm of the duct.

4. A sensor according to claim 3, in which the diffusion surface is generally tubular.

5. A sensor according to any one of the preceding claims, including means for heating gas in a region of the sensor hereby setting up the convection current.

6. A sensor according to claim 5, in which the sensing element is an infra-red absorption sensor, and the infra-red source is arranged also to provide the said means for heating the gas.

7. A sensor according to any one of the preceding claims, further comprising a heater formed around a portion of the duct.

8. A sensor according to any one of the preceding claims, including a fan provided in the duct to provide a forced convection flow.

9. A sensor according to any one of claims 1 to 4, in which the sensing element is a semiconductor or pellistor device.

10. A sensor according to any one of the preceding claims, in which the diffusion surface is formed from a micro-porus plastics material such as polyethylene or PTFE.

## Patentansprüche

1. Gassensor mit einer Diffusionsfläche (4) und einem Sensorelement (1, 3), wobei das Sensorelement (1, 3) getrennt von der Diffusionsfläche (4) untergebracht und mit der Diffusionsfläche (4) durch eine Leitung (2) verbunden ist, dadurch gekennzeichnet, daß die Leitung einen Konvektionsweg für die Aspiration des Sensorelements (1, 3) durch einen Konvektionsstrom, der Gas von der Diffusionsfläche (4) trägt, bildet.

2. Gassensor nach Anspruch 1, wobei die Leitung als geschlossene Schleife ausgebildet ist.

3. Sensor nach Anspruch 2, wobei die Diffusionsfläche sich entlang eines Arms der Leitung erstreckt und das Sensorelement im anderen Arm der Leitung angeordnet ist.

4. Sensor nach Anspruch 3, wobei die Diffusionsfläche generell rohrförmig ist.

5. Sensor nach einem der vorhergehenden Ansprüche mit einem Mittel zu Erwärmen von Gas in einem Bereich des Sensors, um dadurch die Konvektionsströmung zu unterstützen.

6. Sensor nach Anspruch 5, wobei das Sensorelement ein Infrarot-Absorptionssensor ist und die Infrarotquelle ebenfalls das Mittel zum Erwärmen des Gases aufweist.

7. Sensor nach einem der vorhergehenden Ansprüche, zusätzlich mit einer Heizung, die um einen Abschnitt der Leitung ausgebildet ist.

8. Sensor nach einem der vorhergehenden Ansprüche mit einem Propeller, der in der Leitung angeordnet ist, um eine erzwungene Konvektionsströmung zu erzeugen.

9. Sensor nach einem der Ansprüche 1 bis 4, wobei das Sensorelement eine Halbleiter- oder Pellistor-Vorrichtung ist.

10. Sensor nach einem der vorhergehenden Ansprüche, wobei die Diffusionsfläche aus einem mikroporösen Kunststoff, zum Beispiel Polyethylen oder PTFE gebildet ist.

## Revendications

1. Capteur de gaz comprenant une surface (4) de diffusion et un élément capteur (1, 3), l'élément capteur (1, 3) étant logé séparément de la surface (4) de diffusion et relié à la surface (4) de diffusion par un conduit (2), caractérisé en ce que le conduit procure un trajet de convection pour l'aspiration de l'élément capteur (1, 3) par un courant de convection transportant le gaz depuis la surface (4) de diffusion.

2. Capteur de gaz selon la revendication 1, dans lequel le conduit a la forme d'une boucle fermée.

3. Capteur selon la revendication 2, dans lequel la surface de diffusion s'étend le long d'un bras du conduit et l'élément capteur est situé dans l'autre bras du conduit.

4. Capteur selon la revendication 3, dans lequel la surface de diffusion est globalement tubulaire.

5. Capteur selon l'une quelconque des revendications précédentes, incluant un moyen destiné à chauffer le gaz dans une région du capteur en établissant ainsi le courant de convection.

6. Capteur selon la revendication 5, dans lequel l'élément capteur est un capteur à absorption d'infrarouge et la source d'infrarouge est aussi disposée pour constituer ledit moyen destiné à chauffer le gaz.

7. Capteur selon l'une quelconque des revendications précédentes, comprenant en outre un réchauffeur formé autour d'une partie du conduit.

8. Capteur selon l'une quelconque des revendications précédentes, incluant un ventilateur disposé dans le conduit pour créer un courant de convection forcée.

9. Capteur selon l'une quelconque des revendications 1 à 4, dans lequel l'élément capteur est un dispositif à semi-conducteur ou à pellistor.

10. Capteur selon l'une quelconque des revendications précédentes, dans lequel la surface de diffusion est formée à partir d'une manière plastique microporeuse comme du polyéthylène ou du PTFE (polytétrafluoroéthylène).
